(19) Europäisches Patentamt
European Patent Office
Office européen des brevets

(11) EP 1 416 975 B1

# (12) EUROPEAN PATENT SPECIFICATION

(45) Date of publication and mention of the grant of the patent:
**26.01.2005 Bulletin 2005/04**

(21) Application number: **02794578.1**

(22) Date of filing: **07.08.2002**

(51) Int Cl.[7]: **A61L 12/08**, A61L 12/10, A61L 12/12, A61L 2/16, A61L 2/18, A61L 2/23, C11D 3/00, A61K 9/00

(86) International application number:
**PCT/EP2002/008839**

(87) International publication number:
**WO 2003/013621 (20.02.2003 Gazette 2003/08)**

## (54) DISINFECTING AND CLEANSING SYSTEM FOR CONTACT LENSES

SYSTEM ZUR DESINFEKTION UND REINIGUNG VON KONTAKTLINSEN

SYSTEME DE DESINFECTION ET DE NETTOYAGE POUR LENTILLES DE CONTACT

(84) Designated Contracting States:
**AT BE BG CH CY CZ DE DK EE ES FI FR GB GR IE IT LI LU MC NL PT SE SK TR**

(30) Priority: **08.08.2001 US 310893 P**

(43) Date of publication of application:
**12.05.2004 Bulletin 2004/20**

(73) Proprietors:
- **Novartis AG**
  **4056 Basel (CH)**
  Designated Contracting States:
  **BE BG CH CY CZ DE DK EE ES FI FR GB GR IE IT LU MC NL PT LI**
- **Novartis Pharma GmbH**
  **1230 Wien (AT)**
  Designated Contracting States:
  **AT**

(72) Inventors:
- **MOWREY-McKEE, Mary Flowers**
  **Alpharetta, GA 30202 (US)**
- **SILLS, Marzenna, Alicja**
  **Norcorss, GA 30092 (US)**

(74) Representative: **Grubb, Philip William et al**
**Novartis AG,**
**Patent & Trademark Department,**
**Lichtstrasse 35**
**4002 Basel (CH)**

(56) References cited:

| | |
|---|---|
| **EP-A- 0 800 780** | **US-A- 5 169 455** |
| **US-A- 5 419 902** | **US-A- 5 849 291** |

EP 1 416 975 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

BACKGROUND OF THE INVENTION

This invention relates generally to disinfection and cleaning systems for medical devices. In a preferred embodiment, the invention relates to compositions, methods and articles for disinfecting contact lenses.

Contact lenses are known to accumulate dirt, proteinaceous matter, and microorganisms all of which can affect the health of the eye if allowed to accumulate on the lens. Therefore, the lenses must be cleaned and disinfected regularly and preferably daily. To this end, cleaning and disinfecting solutions for use in conjunction with contact lenses have been in use essentially as long as contact lenses have been available to the public. There is considerable diversity in the makeup of the various known solutions, primarily due to the fact that to date no single solution has been found to meet all of the parameters desired for the various types of lenses.

Furthermore, proteins, lipids, and other irritating deposits are not always sufficiently removed by disinfection alone and the lens should be cleaned and rinsed beforehand. This is typically performed by wetting the lens with a sufficient amount of a lens cleaner (such as CIBA Vision® MiraFlow®) and then rubbing the lens with one's fingers and rinsing the lens with saline. The cleaning step is considered a hassle by some consumers and a disinfection system that adequately disinfects and cleans without this step (a "no rub-no rinse" regimen) would offer a great improvement in convenience to the user.

The disinfecting properties of peroxidase enzyme reactions have been well known for many years. Recently, US-A-6,033,662 described an air-activated or oxygen activated disinfection solutions containing a haloperoxidase (i. e., a halide:hydrogen peroxide oxidoreductase, such as myeloperoxidase, eosinophil peroxidase or lactoperoxidase) plus a halide or combination of halides (i.e., chloride, bromide and/or iodide), an oxidase (i.e., a substrate: oxygen oxidoreductase) capable of generating hydrogen peroxide, and a substrate specific for that oxidase. It should be noted that the hydrogen peroxide in these systems is produced from oxygen by a second enzymatic reaction rather than being produced from a peroxy compound such as a perborate or organic peroxide.

Most of the prior art related to decontamination of sensitive biological materials with iodine utilizes iodophors. Iodophors are compositions that utilize povidone-iodine or another chemical complex or contains iodine bound to a high molecular weight polymer. The use of an iodophor increases the absolute concentration of iodine species that are required to inactivate pathogens as compared to a composition of free molecular iodine that does not contain high molecular weight polymers that bind iodine. Iodophors are not the preferred form of iodine for disinfection of sensitive biological material since all forms of iodine can be toxic. A conflict exists between biocidal activity and toxicity with respect to sensitive living cells such as those that comprise the human eye.

Molecular iodine based germicidal compositions for disinfecting pathogenic organisms in or on sensitive biological materials are known. Compositions using a peroxidase, hydrogen peroxide, and an iodide to prepare solutions as ophthalmic medicaments for use directly in the eye are described in US-A-5,849,291. The disinfectant action of such compositions is credited to the *in situ* generation of low levels of molecular iodine.

It is desirable to provide a single solution for simultaneously disinfecting and cleaning contact lenses, since such a system would be both practical and economical. It is also desirable to provide a composition in the form of a dry powder or tablet that, upon addition of water or aqueous saline, will afford an activated solution. The redox compositions of the prior art are chemically complex and the need for careful control of the instant concentrations of the various products and by-products of these systems is recognized by those skilled in the art.

US-A-5,756,044 to *Mowrey-McKee, et al.* discloses a peroxidase/ perborate/iodide system in a single tablet. However, it has been found that the one-part formulations of the peroxidase/peroxide/iodide systems of the prior art are unstable when such formulations include a perborate to generate the required hydrogen peroxide in conjunction with an alkali metal iodide and horseradish peroxidase. The observed instability of these systems has been attributed to direct and premature reaction of the perborate with the iodide thus producing the various iodine species in uncontrolled concentrations.

Furthermore, the pH of the resulting solutions must be controlled in order to prevent imparting an undesirable yellow color to the solution. The formulations of the prior art would allow fluctuations in pH, which resulted in the discoloration of the lens. While the discoloration eventually subsides, the lens is unpleasant to the consumer and unusable for some time.

Accordingly, there is a need for improved cleaning and disinfecting solutions which are compatible for use with most types of contact lenses while maintaining both a high level of antibacterial activity and low order of toxicity to eye tissue. It is further desirable to provide a stable system that affords a convenient to use iodine based contact lens cleaning and disinfecting solution.

## SUMMARY OF THE INVENTION

Accordingly, a method is provided using a hydrogen peroxide/iodide/peroxidase system useful for disinfecting ophthalmic devices such as contact lenses. More specifically the invention pertains to a method in which a first composition comprising a peroxidase enzyme and an iodide salt is provided and a second composition comprising a solution of a source of hydrogen peroxide is also provided. Just prior to use, said first composition is combined with said second composition to provide an activated disinfecting/cleaning solution. The ophthalmic device is effectively disinfected and cleaned by contacting said ophthalmic device with said active disinfecting/cleaning solution.

The present invention also provides a disinfecting and/or cleaning system for ophthalmic devices such as contact lenses. The system is comprised of a source of hydrogen peroxide, an iodide salt, and a peroxidase enzyme. In this system the source of hydrogen peroxide and the iodide components are kept separated until just prior to utilization of the system, and thus the premature reaction of peroxide and iodine is prevented. The two-part systems of this invention have greatly improved stability compared to comparable one-part systems. Furthermore the two-part systems of this invention can be formulated with additional ingredients such as hydrogen peroxide stabilizers to afford products with excellent shelf life.

## DETAILED DESCRIPTION OF THE PREFERRED EMBODIMENT

In one embodiment of this invention a tablet is provided comprising an iodide salt and a peroxidase enzyme and independently provided is a saline solution containing a perborate as a source of hydrogen peroxide. Just prior to the use the tablet is dissolved in the saline/perborate solution thus providing an activated disinfecting solution into which the contact lens, or other ophthalmic device be disinfected, is introduced.

The disinfecting methods and systems of the present invention are effective against a wide spectrum of microorganisms, including but not limited to *Staphylococcus aureus, Pseudomonas aeruginosa, Serratia marcescens, Candida albicans,* and *Fusarium solani.*

A disinfecting solution is generally defined as a contact lens care product containing one or more active ingredients (for example, anti-microbial agents and/or preservatives) in sufficient concentrations to destroy harmful microorganisms on the surface of a contact lens within the recommended minimum soaking time. The recommended minimum soaking time is included in the package instructions for use of the disinfecting solution. The present solution, in combination with its container or bottle and packaging, including instructions for use, may be considered a novel and improved kit, package, or system for the care of contact lenses.

The term "soft lens" means a lens having a proportion of hydrophilic repeat units such that the water content of the lens during use is at least 20% by weight. The term "soft contact lens" as used herein generally refers to those contact lenses that readily flex under small amounts of force. Typically, soft contact lenses are formulated from polymers having a certain proportion of repeat units derived from hydroxyethyl methacrylate and/or other hydrophilic monomers, typically crosslinked with a crosslinking agent. In contrast, conventional "hard contact lenses," which cover only a part of the cornea of the eye, usually consist of poly(methyl methacrylate) crosslinked with ethylene glycol dimethacrylate or the like, and conventional rigid gas permeable lenses (RGP) typically consists of monomers containing silicon that result in a more oxygen-permeable material.

By the term "ophthalmically safe" with respect to a contact lens solution is meant that a contact lens treated with the solution is safe for direct placement on the eye without rinsing, that is, the solution is safe and sufficiently comfortable for daily contact with the eye via a contact lens. An ophthalmically safe solution has a tonicity and pH that is compatible with the eye and comprises materials, and amounts thereof, that are non-cytotoxic according to international !SO standards and U.S. FDA regulations.

The term "compatible with the eye" means a solution that may be in intimate contact with the eye for an extended period of time without significantly damaging the eye and without significant user discomfort.

The term "disinfecting solution" means a solution containing one or more microbiocidal compounds, that is effective for reducing or substantially eliminating the presence of an array of microorganisms present on a contact lens, which can be tested by challenging a solution or a contact lens after immersion in the solution with specified inoculums of such microorganisms. The term "disinfecting solution" as used herein does not exclude the possibility that the solution may also be useful for a preserving solution or that the disinfecting solution may additionally be useful for daily cleaning, rinsing, and storage of contact lenses.

The term "cleaning" means that the solution contains one or more active ingredients in sufficient concentrations to loosen and remove loosely held lens deposits and other contaminants on the surface of the article to be cleaned. While not necessary with the present invention, a user may wish to use the solutions of the present invention in conjunction with digital manipulation (for example, manual rubbing of the lens with a solution) or with an accessory device that agitates the solution in contact with the lens, for example, a mechanical cleaning aid.

A solution that is useful for cleaning, chemical disinfection, storing, and rinsing an article, such as a contact

lens, is referred to herein as a "multi-purpose solution." Such solutions may be part of a "multi-purpose solution system" or "multi-purpose solution package." The procedure for using a multi-purpose solution, system, or package is referred to as a "multifunctional disinfection regimen." Multi-purpose solutions do not exclude the possibility that some wearers, for example, wearers particularly sensitive to chemical disinfectants or other chemical agents, may prefer to rinse or wet a contact lens with a another solution, for example, a sterile saline solution prior to insertion of the lens. The term "multi-purpose solution" also does not exclude the possibility of periodic cleaners not used on a daily basis or supplemental cleaners for removing proteins, for example enzyme cleaners, which are typically used on a weekly basis.

In another embodiment of this invention a tablet is provided comprising an iodide salt and a peroxidase enzyme, and independently provided is a saline solution containing hydrogen peroxide or a source of hydrogen peroxide and a hydrogen peroxide stabilizer. This embodiment thus provides a two-part system with improved shelf-life. Just prior to the use the tablet is dissolved in the saline/perborate solution thus providing an active disinfecting solution into which the contact lens, or other ophthalmic device be disinfected, is introduced.

In accordance with the present invention free molecular iodine is generated *in situ* as a microbicidal agent by the peroxidase catalyzed reaction of an iodide salt and hydrogen peroxide. During the course of this redox reaction the hydrogen peroxide is reduced to water with the concomitant oxidation of the iodide anion to molecular iodine. The instant solution generated during the course of this reaction contains; (1) the enzyme generated components including molecular iodine, (2) iodide at levels that are reduced from the initial concentration, (3) peroxide at levels that are reduced from the initial concentration, and (4) the peroxidase enzyme at the initial concentration.

Any peroxidase enzyme that catalyzes the hydrogen peroxide oxidation of iodide anion to molecular iodine may be used in the present invention. Such peroxidase can be obtained from a variety of sources and their properties vary as a function of the source. Particularly useful are those peroxidases that fall under the International Union of Pure and Applied Chemistry (IUPAC), Enzyme Commission (E.C.) classification No.1.11.1.7. Practical considerations of cost and stability favor horseradish peroxidase (HRP) as the preferred peroxidase within the E.C. No. 1.11.1.7 classification. The molecular weights of peroxidases range from 12,000 Daltons for NADH peroxidase to 149,000 Daltons for myeloperoxidase. Horseradish peroxidase has a molecular weight of about 40,000 Daltons. A list of peroxidases suitable for use in this invention includes but is not limited to horseradish peroxidase, microbial peroxidase, GD peroxidase, peroxidase 540, lactoperoxidase, myleroperoxidase, and soybean peroxidase.

In the iodine-producing redox reaction of this invention an iodide anion serves as a substrate for a peroxidase enzyme. This reaction is put forth in more detail in US-A-4,588,586 to *Kessler, et al.,* incorporated herein by reference. Suitable sources of iodide anion include sodium iodide and potassium iodide as well as other iodide salts. The simple salts of iodide have the advantage of being relatively inexpensive. Iodide anion must be dissolved in an aqueous environment and contacted with the peroxidase enzyme in the presence of hydrogen peroxide. The concentration of iodide anion used for this invention ranges from about 0.10 to about 5.0 mg/7 mL. The preferred range for iodide anion lies between about 0.2 and about 1.5 mg/7 mL and is a function of the pH and ionic strength of the solution in addition to the peroxide concentration.

The preferred oxidant of this invention is hydrogen peroxide. Therefore, any material which acts as a source of hydrogen peroxide when admixed in an aqueous environment is suitable for the present invention. The terms "source of peroxide" and "source of hydrogen peroxide" for purposes of the present invention and as used herein shall mean any material alone or in combination which can serve as precursors for hydrogen peroxide such materials including metal peroxides, percarbonates, persulphates, perphosphates, peroxyesters, urea peroxide, peroxyacids, alkylperoxides, acylperoxides and perborates. Alternatively, methyl peroxide can also be used as a source of hydrogen peroxide. Mixtures of two or more of these substances can also be used. The preferred sources of hydrogen peroxide include sodium perborate, sodium peroxide, and urea peroxide. Hydrogen peroxide concentrations from about 10 ppm to about 2500 ppm are useful in the present invention. Therefore, sources of hydrogen peroxide that generate hydrogen peroxide from about 10 ppm to about 2500 ppm are useful in the present invention. The preferred initial concentration for hydrogen peroxide in the final disinfecting composition is between about 10 and 1000 ppm, preferably an amount from 10 to 100 ppm. Because the disinfection in the present invention is performed by iodide, it is not necessary to have microbicidal levels of hydrogen peroxide in the solution. Because hydrogen peroxide is irritating to ocular tissues, the concentration of peroxide is kept as low as possible, preferably at a level at which the peroxide is insufficient to function of itself, as a bactericidal agent in the solution.

As stated above, peroxide compounds are irritating to ocular tissue. Thus, it is preferable that during the sterilization of the lenses by the methods and systems of this invention, a substantial percentage of the peroxide is consumed during the reaction. By "substantial percentage" it is meant that the level remaining in the solution immediately prior to insertion of the lens into the eye is not irritating to ocular tissue according to the well-recognized Draize scale.

The stabilizers useful in the present invention are any of the known stabilizers of peroxy compounds including phosphonates, phosphates, stannates, etc. However, physiologically compatible water-soluble salts of phosphonic acids are preferred. Within this preferred group are

(a) compounds of the formula

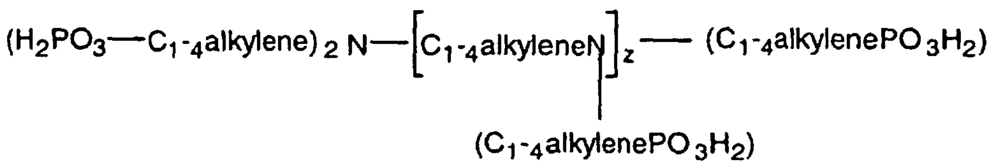

(I)

wherein z is an integer from 0-3, and water-soluble salts thereof: and

(b) compounds of the formula

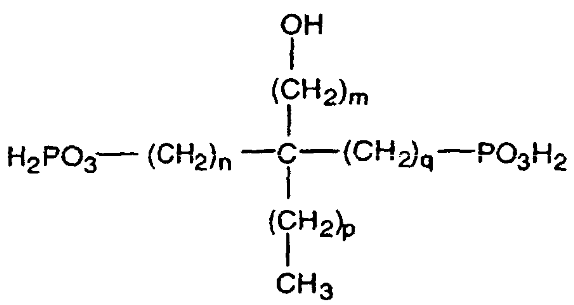

(II)

wherein each of n, m, p, and q is independently 0-4, and water-soluble salts thereof. Highly preferred within formula I are compounds wherein z is 2 and compounds wherein each $C_{1-4}$ alkylene group is $C_1$ or $C_2$. Most preferred within formula I is diethylene triamine penta(methylene-phosphonic acid) and the physiologically compatible water-soluble salts thereof, marketed by Solutia, Inc. (formerly Monsanto) under the name DEQUEST™ 2060. Highly preferred within formula If are compounds wherein n, m, p and q are each 0 or 1, most preferably zero, or a physiologically compatible water-soluble salt thereof. Such a compound is marketed by Solutia, Inc. (formerly Monsanto) under the name DEQUEST 2010.

Physiologically compatible salts of the compounds of formulae I and II include, for example, water soluble salts with conventional pharmaceutically acceptable cationic moieties, including the alkali metal, e.g. sodium, potassium, alkaline earth metal, e.g. calcium, ammonium and amine cations. Suitable amine salts include, for example, mono-, di-, and tri-lower alkyl amines, e.g. methylamine, ethylamine, diethylamine, triethylamine, dimethylamine, trimethylamine, propylamine, and the like; and mono-, di-, and tri-lower hydroxyalkyl amines, e.g. ethanolamine, diethanolamine, triethanolamine, glucamine, 2-hydroxypropylamine, and the like. By "lower" in the context of an alkyl group is meant an alkyl group having up to 6 carbon atoms, preferably up to 4 carbon atoms. If desired, additional conventional stabilizers may be employed in conjunction with those of formulae I or II or combinations thereof in accordance with the present invention. Suitable conventional stabilizers include: water soluble stannates, such as an alkali metal or ammonium stannate, for example sodium stannate, alone or in combination with a water soluble phosphate, polyphosphate or etaphosphate salt, such as an alkali metal or ammonium salt thereof; or an amino polycarboxylic acid chelating agent, such as ethylene diamine tetraacetic acid, nitrilo triacetic acid or a water soluble salt thereof, such as an alkali metal or ammonium salt, especially the sodium salt, or mixtures thereof.

Still further peroxy stabilizers which may be used in the invention include a peroxide stabilizer selected from glycerin, polyvinyl alcohol having a molecular weight in the range of about 2,000 to about 150,000 (as long as water soluble) and being at least 70% hydrolized, propylene glycol, polyacrylic acid having a molecular weight of about 2,000 to about 100,000, diethylene glycol, and sodium hexamethaphosphate sodium polyphosphate (available from FMC under the name HEXAPHOS™).

The above stabilizers can be used in almost all situations previously mentioned to which the invention is applicable. However, when the solution is to come in contact with a hydrogel soft contact lens, stannate stabilizers are to be avoided, as they tend to "cloud" the lens material. Preferably, the concentration of the stabilizer of formula I or salt thereof is present in the stabilized composition in an amount between about 0.006 and about 0.02% by weight of the composition, and most preferably between about 0.006 and about 0.0120% by weight of the composition. The stabilizer of formula II is present per 100 g of solution in an amount of at least about 0.024 m mole (50 ppm), preferably 0.039 m mole (80 ppm) up to about 0.34 m mole (700 ppm) more preferably 0.049 m mole (100 ppm) up to about 0.29

m mole (600 ppm), most preferably 0.073 m mole (150 ppm) to about 0.19 m mole (400 ppm). The amounts in parentheses are for DEQUEST 2010 which has a molecular weight of 206. Other stabilizers of formula II should be present in molar equivalents thereto. The stabilizers other than those of formula I and II are employed in a physiologically tolerable amount, e.g. about 20 ppm to about 1000 ppm, preferably in an amount of at least 0.054 m mole (50 ppm), more preferably 0.087 m mole (80 ppm) to about 1.09 m mole (1000 ppm), still more preferably from about 0.109 m mole (100 ppm) to about 0.87 m mole (800 ppm), most preferably about 0.22 m mole (200 ppm) to about 0.65 m mole. The diethylene triamine penta(methylene phosphonic acid) has a molecular weight of 206. Other stabilizers of formula II should be present in molar equivalents thereto.

Also, there may be present in the solutions according to the present invention one or more conventional, substantially inert, physiologically acceptable tonicity enhancing agents. Suitable such agents include, for example, alkali metal halides, phosphates, hydrogen phosphate, and borates. Preferred are sodium chloride, sodium phosphate monobasic and sodium phosphate dibasic. The function of such tonicity enhancing agents is to assure approximate physiologic tonicity, e.g. substantially equivalent in tonicity to a 0.9% by weight aqueous sodium chloride solution, to the solution which is instilled in the eye or to help assure such tonicity upon dilution if dilution is necessary prior to contact with the eye due to peroxide content as indicated above. The tonicity of common ocular solutions is about 200 -300 milliOsmoles / L.

Any buffering agent which has the capacity to control the pH to within reasonable limits is suitable for use with this invention provided that such a buffering agent is compatible with the other ingredients of the system. For example, it is important that the buffering agent not react with chemically active components such as peroxides or chelating stabilizers. A list of suitable buffers for the present invention includes, but is not limited to, inorganic or organic bases, preferably basic acetates, phosphates, borates, citrates, nitrates, sulfates, tartrates, lactates, carbonates, bicarbonates, TRIS, BIS-TRIS propane, Goode buffers, and mixtures thereof, more preferably basic phosphates, borates, citrates, tartrates, carbonates, bicarbonates and mixtures thereof. It is important to control the pH to > 6.0 to prevent unacceptable coloration of the contact lens by iodine. Accordingly, the buffering agent is preferably present in an amount effective to provide buffer capacity to prevent a decrease in pH of the solution to below about 6.0 while the substrate is being disinfected. More preferably, the solution has buffer capacity to prevent a decrease in pH of the solution to below about 6.5 while the substrate is being disinfected.

The buffer component preferably includes one or more phosphate buffers, for example, combinations of monobasic phosphates, dibasic phosphates, and the like. Particularly useful phosphate buffers are those selected from phosphate salts of alkali and/or alkaline earth metals. Examples of suitable phosphate buffers include one or more of sodium dibasic phosphate ($Na_2HPO_4$), sodium monobasic phosphate ($NaH_2PO_4$), and potassium monobasic phosphate ($KH_2PO_4$).

Maintaining a pH above about 6.0 is particularly important when the substrate to be disinfected is a soft contact lens. Contact lenses are typically made from a polymer of an organic compound such as a methacrylic acid derivative, acrylamide derivative, or N-vinyl pyrrolidone. Iodine molecules are easily adhered to the matrix of a lens at the time of disinfection, thus discoloring the lens.

Adequate buffer capacity is also particularly important when the peroxidase/iodide component of the present system is in tablet form, especially in an effervescent tablet form. While the tablet is preferably uniform in texture, tablets may contain particles and other inconsistencies in texture that cause the components thereof to dissolve into the aqueous solution at different times during the dissolution of the tablet. Such unpredictable solubilizing of the components may cause undesired swings in pH, thus causing the lens discoloration noted above. As discussed below, effervescent tablets contain an acid-base effervescent couple, which can cause pH changes if the acid and/or base enter the solution at different rates before reacting with each other.

A reducing agent is preferably employed to reduce the iodine back to iodide following disinfection to result in a clear, nearly colorless solution. When the reducer reduces iodine molecules after disinfection, they are converted into iodide anion ($I^-$), which is colorless and safe and has almost no adsorption power to the lens matrix. Sodium sulfite, sodium hydrogen sulfite, sodium thiosulfate, ascorbic acid, and sodium ascorbate are particularly preferred because they effectively reduce iodine molecules remaining in a soft contact lens after disinfection. Other hydroxycarboxylic acids or salts thereof may be employed; for example, citric, malic, tartaric, or ascorbic acid, and/or a salt, especially the sodium, potassium, or calcium salt, of such an acid. Of these, citric acid, sodium citrate, and ascorbic acid are especially preferable. Additionally, a number of other reducing agents can be successfully employed in the present invention, including, but not limited to, reducing sugars, (e.g. lactose and glucose); amino acids (e.g., tyrosine); and imidazole.

Tablets containing the peroxidase enzyme and an iodide salt are preferred to be effervescent in order to reliably conduct a treatment of the lens. Accordingly, the tablet preferably includes an acid and a base which form an effervescent couple that react in solution to generate a stream of $CO_2$ bubbles when the components dissolve to aid dissolution and break up of the formulation. The base should preferably be sodium bicarbonate, and the acid is preferably a weak organic acid such as tartaric, citric or malic acid. The acid and base should be present in amounts that

are approximately equimolar. The $CO_2$ bubbles formed by reaction between the acid and base can aid the dispersion of any other components in the tablet and solution, and can thereby decrease the time needed to disinfect the lens. The tablets may further contain, for example, a lubricant, or a disintegrating agent, ordinarily used in tablet preparation.

Additionally, the components of the disinfecting / cleaning systems of the present invention may contain additional ingredients such as lytic enzymes, chelating agents, surfactants, viscosity modifying agents, bulking agents, and lubricants. Such additives can aid in tablet preparation, lens cleaning efficacy and disinfecting efficacy.

Various lytic enzymes are useful in this invention to enhance the cleaning efficacy of the system. Such enzymes include both naturally occurring enzymes and protein engineered enzymes prepared by any of the various recombinant DNA techniques. These useful classes of enzymes include proteases, lipases, amylases, and mucin-degrading enzymes. A list of suitable additional enzymes includes, but is not limited to, pancreatic lipase, phospholipase, subtilisin, trypsin, lysozyme, hyaluronidase, and the like.

Suitable surfactants include, but are not limited to tyloxapol, which is 4-(1,1,3,3-tetramethylbutyl)phenol polymer with formaldehyde and oxirane; poloxamers (PLURONIC™ and PLURONIC -R™) which are nonionic surfactants consisting of block copolymers of propylene oxide and ethylene oxide; octoxynol or octyphenoxy polyethoxyethanol prepared by reacting isooctylphenol with ethylene oxide; poloxamine which is a block copolymer derivative of ethylene oxide and propylene oxide combined with ethylene diamine; and nonoxynol nonionic surfactant mixtures prepared by reacting nonylphenols with ethylene oxide. The surfactants can be employed in amounts ranging from about 0.0001 to about 20% by weight, preferably from about 0.005 to about 5.0% by weight, more preferably from about 0.025 to about 1.0 % by weight.

The preparation of tablets of the compositions of this invention may benefit from the inclusion of certain lubricants and bulking agents that are well know in the art of tablet preparation. Common lubricants include polyethylene glycol (PEG), sodium benzoate, and calcium stearate. However, PEG should not be used as plating out of components has been observed when PEG is used. Starting at the bottom of the disinfection container, a series of reddish-brown plates precipitate out of solution. Contact of these plates with the lens caused significant discoloration of the lens, which did not dissipate until about 24 hours. This problem appears to be exacerbated when the peroxide containing solution lacks sufficient buffer capacity to maintain a pH above about 6.0. Thus, the tablet of the present invention is preferably free of PEG. Because sodium benzoate also exhibits preservative efficacy, it is the most preferred lubricant. While any of the common bulking agents may be employed in this invention, sugars and similar carbohydrates are preferred and lactose is particularly preferred.

Suitable viscosity modifying agents include, but are not limited to lecithin or the cellulose derivatives such as hydroxymethyiceilulose, hydroxypropylcellulose and methylcellulose in quantities similar to those used for the surfactants.

It should be emphasized here that the invention is also applicable beyond the field of ophthalmic device disinfection and may be used anywhere that a disinfecting solution treatment would be useful such as other medical devices, provided only that the material treated is not adversely affected by the composition components. For these purposes the compositions need not be ophthalmic device compatible or even pharmaceutically acceptable. The only important feature in such a case is that the system is comprised of a source of hydrogen peroxide, an iodide salt, and a peroxidase enzyme; and that the source of hydrogen peroxide is kept separated from the iodide component until just prior to utilization of the system. Medical devices include ophthalmic, dental, surgery, laboratory equipments and tools.

Methods for treating an ophthalmic device such as a contact lens using the herein described disinfecting/ cleaning compositions are included within the scope of the invention. Such methods comprise contacting an ophthalmic device with such a disinfecting/cleaning system at conditions effective to provide the desired treatment to the ophthalmic device. Each of the specific disinfecting/cleaning formulations described above may be employed in these methods.

The method for disinfecting and cleaning of the present invention can be applied to all of known hydrous soft contact lenses. Stated more specifically, a lens is held in a basket fixed to the cap of a capped vial after the vial is filled with an aqueous solution, the first and second formulations are added and the cap having the lens fixed in the basket is closed.

The preferred lens basket for use with the presently claimed method and system is the treatment system disclosed in US-A-5,756,044 to *Mowrey-McKee, et al.* Specifically, for example, a tablet including a peroxidase and an iodide salt, in addition to an effervescent couple may be formed. A saline solution containing sodium perborate may be placed in the present lens treatment container along with the tablet. Contact lenses may be placed in the present lens-retaining means and the lenses-retaining means immersed into the saline solution in the container. As the tablet dissolves, the disinfection components release and travel primarily upward with the effervescent bubbles through the central tubular channel, then downwardly between the container walls and through the lens- retaining means, thereby bathing the lens on both convex and concave surfaces.

The contacting temperature is preferred to be in the range of about 0°C to about 100°C, and more preferably in the range of about 10°C to about 60°C and still more preferably in the range of about 15°C to about 37°C. Contacting at or about ambient temperature is very convenient and useful. The contacting preferably occurs at or about atmospheric pressure. The contacting preferably occurs for a time in the range of about 5 minutes or about 1 hour to about 12 hours or more.

Typical non-ophthalmic device disinfecting applications for which such compositions are useful include: lens case cleaner and disinfectant, topical medical composition, industrial disinfectant, laboratory disinfectant, dental and medical equipment disinfectant, acne cleaning and disinfecting treatments, insect bite disinfection, for minor skin itching and rashes and wound healing applications. It is also suitable as a rapid in-office contact lens disinfecting/cleaning regimen.

The following examples are presented for illustrative purposes and are not intended to in any way limit the scope of this invention.

EXAMPLE I

This example presents typical formulations and methods of preparation of each component of a two-part system of the present invention. Table I provides compositions for the preparation of peroxidase / iodide tablets, while Table II provides a composition for a buffered saline / peroxide solution.

TABLE 1

| (tablet formulations) | | | | | | |
|---|---|---|---|---|---|---|
| ingredient | A | B | C | D | E | F |
| horseradish peroxidase (units/tablet) | 300.0 | 0.0 | 0.0 | 0.0 | 0.0 | 0.0 |
| microbial peroxidase (mg/tablet) | 0.0 | 2.0 | 2.0 | 0.50 | 1.0 | 0.50 |
| Trypsin (mg/tablet) | 0.0 | 1.00 | 0.0 | 0.0 | 0.0 | 0.0 |
| Subtilisin (mg/tablet) | 8.0 | 0.0 | 8.0 | 8.0 | 8.0 | 4.0 |
| Lipase (mg/tablet) | 2.0 | 2.0 | 2.0 | 2.0 | 2.0 | 2.0 |
| sodium benzoate (mg/tablet) | 7.4 | 7.4 | 7.4 | 7.4 | 7.4 | 7.4 |
| potassium iodide (mg/tablet) | 0.3 | 0.3 | 0.3 | 0.3 | 0.3 | 0.3 |
| lactose monohydrate (mg/tablet) | 63.0 | 69.0 | 62.0 | 63.0 | 62.0 | 66.0 |
| citric acid (mg/tablet) | 33.0 | 33.0 | 33.0 | 33.0 | 33.0 | 33.0 |
| potassium carbonate (mg/tablet) | 47.0 | 47.0 | 47.0 | 47.0 | 47.0 | 47.0 |
| weight of tablet (mg) | 160.70 | 161.70 | 161.70 | 161.20 | 160.70 | 161.20 |

Individual tablets are prepared from the individual formulations A-F shown in Table I using a conventional laboratory tablet press.

TABLE II

| (saline/perborate solution) | |
|---|---|
| Ingredient | Quantity (gram/liter) |
| sodium chloride | 1.60 |
| sodium borate decahydrate | 0.54 |
| boric acid | 10.00 |
| sodium perborate tetrahydrate | 0.27 |
| DEQUEST 2060 | 0.06 |
| purified water | QS to 1.00 Liter |
| note: DEQUEST 2060 is the trade name for a diethylene triamine penta(methylene-phosphonic acid) sodium salt. | |

A saline/perborate solution is prepared by combining and agitating the ingredients shown in Table II until all components are dissolved. The solution pH is adjusted to pH 7.0 ± 0.1 with either 5N hydrochloric acid or 5N sodium

hydroxide as required. The osmolality is adjusted to 220 ± 10 mOsm with either sodium chloride or purified water as required. If the perborate concentration is lower than 249 ppm, the concentration is adjusted to be from 249 to 300 ppm by the addition of sodium perborate. If the perborate concentration is higher than 300 ppm the batch is discarded. The resultant solution is sterilized by filtration through a 0.22 micron filter.

EXAMPLE II

This example demonstrates the use of the systems prepared from the formulations of Table I and Table II. Just prior to use the individual tablets A-F of Table I are dissolved in 7.0 ml aliquots of the saline/perborate solution of Table II. Contact lenses (CIBA Vision type 4 soft lens) are inoculated with *Candida albicans* and such a lens is introduced into each solution.

After 1 hr at room temperature the log reduction of the *Candida albicans* is determined using standard microbiological methods. Ten replicates are performed with each formulation A-F and the results are presented in Table III.

TABLE III

| | log reduction *Candida albicans* (1.0 hr exposure) | | | | | |
|---|---|---|---|---|---|---|
| replicate | A | B | C | D | E | F |
| 1 | 2.9 | TK | TK | TK | TK | 3.8 |
| 2 | 3.1 | TK | TK | 2.2 | 1.2 | TK |
| 3 | TK | TK | TK | TK | 3.3 | TK |
| 4 | 2.9 | 4.5 | TK | 3.7 | TK | TK |
| 5 | 2.0 | TK | TK | 3.7 | TK | TK |
| 6 | 3.0 | TK | TK | TK | TK | TK |
| 7 | 3.1 | TK | TK | 2.9 | 3.5 | TK |
| 8 | TK | TK | TK | 4.8 | TK | 4.9 |
| 9 | 2.8 | TK | 4.3 | TK | 4.1 | TK |
| 10 | 2.3 | TK | TK | 4.5 | TK | TK |
| note: TK = total kill | | | | | | |

These data demonstrate the excellent disinfecting efficacy of the system of this invention. Additionally it is observed visually that the lenses thus treated retain a color near that of a new lens.

EXAMPLE III

This example tests the efficacy of the systems prepared from the formulations of the present invention pursuant to ISO 14729 (First Edition, April 15, 2001). Vifilcon A lenses were inoculated with microorganisms in organic soil and placed into the cup of US-A-5,756,044 with a tablet of either Formulation G or Formulation H and a solution of Table II. Formulation G was similar to that of Tablets C - F of Table I, but with 4 mg peroxidase and 0.45 mg KI. Formulation H was similar to that of Tablet C - F of Table I, but with 3.1 mg peroxidase and 0.65 mg KI. To compensate for the additional iodide and peroxidase, the lactose content of the tablet was reduced to yield a tablet of similar weight. Lenses were removed after 4 hours and cultured on growth media. The solution was filtered and the filter cultured on growth media. Twelve lenses were evaluated for each organism per tablet formulation. The passing criteria for a "no rub - no rinse" regimen is < 10 cfu/lens + solution combo (cfu means colony forming units).

TABLE IV

| | **Tablet G** | **Tablet H** |
|---|---|---|
| *Candida atbicans* | 0 - 1 cfu/lens<br>0 - 11 cfu/sotution<br>Mean 1.4 cfu/lens +solution | 0 - TNTC cfu/lens<br>0 - TNTC cfu/solution<br>Mean 2.2 cfu/lens +solution |
| *Fusarium solani* | 0 - 1 cfu/lens | 0 - 2 cfu/lens |

TABLE IV (continued)

| | Tablet G | Tablet H |
|---|---|---|
| | 0 - 3 cfu/solution<br>Mean 0.33 cfu/lens +solution | 0 - 6 cfu/solution<br>Mean 1.7 cfu/lens +solution |
| *Pseudomonas aeruginosa* | 0 cfu/lens<br>0 cfu/solution<br>Mean 0 cfu/lens +solution | 0 cfu/lens<br>0 cfu/solution<br>Mean 0 cfu/lens +solution |
| TNTC = Too Numerous to Count. This is because one tablet tested was a "dud." | | |

These data demonstrate the excellent disinfecting efficacy of the system of this invention and is a "pass" under the relevant ISO standard for a "no rub - no rinse" regimen.

EXAMPLE IV

This example demonstrates the safety of the systems prepared from the formulations of the present invention. Just prior to use, the individual tablets H and G were dissolved in 7.0 ml aliquots of the saline/perborate solution of Table II. Contact lenses (CIBA Vision vifilcon A) were cleaned with the resulting solution as per the invention. At the height of the reaction (about 30 minutes), each lens was taken out, and put on six eyes of house rabbits without rising on the first day. On the second day, the same operation as described above was repeated, and the eye irritancy of this system was evaluated each day. The evaluation was based on the observed score according to the criteria of Draize (ISO 9394) and on the corneal staining density with fluorescein immediately after removal of the lens from tested eyes on each day.

The criteria of the Draize method are shown in Table V.

TABLE V

| Site | Degree of Reaction | |
|---|---|---|
| Comea | (A) Opacity - Degree of Density | 0 |
| | • Transparent, no opacity<br>• Scattered or diffuse area, details of iris clearly visible<br>• Easily discernible translucent areas, details of iris slightly obscured<br>• Opatesent areas no details of iris visible, size of pupil barely discerible<br>• Opaque, iris invisible<br>(B) Area of Comea<br>• 0~¼<br>• ¼~½<br>• ½~¾<br>• ¾~4/4 | <br>1<br>2<br>3<br>4<br><br>1<br>2<br>3<br>4 |
| Iris | (A) Values | |
| | • Normal<br>• Folds above normal, congestion, swelling circumcorneal injection (any or all of these or combination of any thereof), iris still reacting to light sluggish reaction is positive)<br>• No reaction to light, hemorrhage, gross destruction (any or all of these.) | 0<br>1<br><br>2 |
| Conjunctiva | (A) Redness of Palpebral and Bulbar Conjunctiva | |
| | • Vessels normal<br>• Vessels definitely injected above normal<br>• More diffuse, deeper crimson red, individual vessels not clearly discernible<br>• Diffuse beefly red<br>(B) Chemosis<br>• No swelling<br>• Any swelling above normal (includes nictitating membrane) | 0<br>1<br>2<br>3<br><br>0<br>1 |

TABLE V (continued)

| Site | Degree of Reaction | |
|---|---|---|
| | • Obvious swelling with partial eversion of lids | 2 |
| | • Swelling with lids about half closed | 3 |
| | • Swelling with lids about half closed to completely closed | 4 |
| | (C) Discharge | |
| | • No discharge | 0 |
| | • Any amount different from normal (does not include amount observed in inner thus of normal) | 1 |
| | • Discharge with moistening of the lids and hairs just adjacent to lids | 2 |
| | • Discharge with moistening of the lids and hairs, and considerable area around the eye | 3 |

Evaluation points were determined by the symptoms and observations of the eye tissues and the safety of the formulation was evaluated by the total of these. Calculation of The Total of the evaluation points:

$$\text{Total Points}=[\text{cornea}:A\times B\times 5+\text{iris}:A\times 5+\text{conjunctiva}:(A+B+C)\times 2]$$

Evaluation:

0 to 5 points: no irritating
5 to 15 points: slightly irritating
15 to 30 points: irritating
30 to 60 pints: moderately irritating
60 to 80 points: moderately to strongly irritating
80 to 110 points: strongly irritating

Very minimal redness was observed initially and no cumulative effects were observed on the second day. As minimal redness is frequently observed following redness on rabbit eyes, all lenses treated with the system of the present invention were deemed to be non-irritating - even when they were not rinsed after disinfection and prior to insertion into the eye.

## Claims

**1.** A system for disinfecting and cleaning a contact lens comprising:

(a) a first composition comprising a peroxidase enzyme and an iodide salt;
(b) a second composition comprising a source of hydrogen peroxide in an aqueous solution;

wherein said first composition is in tablet form;
wherein said tablet comprises sodium benzoate and lactose; and
wherein said first composition further comprises a buffering agent in an amount effective to provide buffer capacity to prevent a decrease in pH to below about 6.0 when said first composition is combined with said second composition.

**2.** The system of claim 1 wherein said first and second composition are kept separated until just prior utilization of said system.

**3.** The system of Claim 1, wherein said buffering agent is selected from the group consisting of acetates, phosphates, borates, citrates, nitrates, sulfates, tartrates, lactates, carbonates, bicarbonates, TRIS, BIS-TRIS propane, Goode buffers, and mixtures thereof.

**4.** The system of Claim 1, wherein said first composition comprises an amount of iodide salt sufficient to provide a concentration of iodide anion from about 0.10 to about 5.0 mg/7 mL when combined with said second composition.

**5.** The system of Claim 4, wherein said first composition comprises an amount of iodide salt sufficient to provide a concentration of iodide anion from about 0.2 to about 1.5 mg/7 mL when combined with said second composition.

**6.** The system of Claim 1, wherein said tablet further comprises an acid and a base which form an effervescent couple that react when exposed to said second composition.

**7.** The system of claim 1, wherein said peroxidase enzyme is selected form the group consisting of horseradish peroxidase, microbial peroxidase, GD peroxidase, peroxidase SP 540, actoperoxidase, myleroperoxidase, and soybean peroxidase.

**8.** The system of claim 1, wherein said source of hydrogen peroxide is selected from the group consisting of hydrogen peroxide, sodium perborate, sodium peroxide, and urea peroxide.

**9.** The system of claim 1, wherein said source of hydrogen peroxide provides hydrogen peroxide in an amount from 10 ppm to 1000 ppm, preferably in amount from 10 ppm to 100 ppm.

**10.** The system of claim 1, wherein said second composition further comprises a hydrogen peroxide stabilizer.

**11.** The system of claim 10, wherein said hydrogen peroxide stabilizer is selected from the group consisting of

(a) compounds of the formula

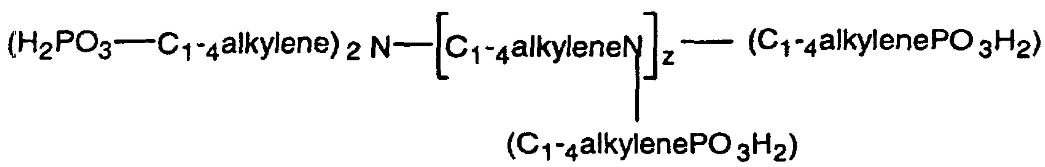

(I)

wherein z is an integer from 0-3, and water-soluble salts thereof; and
(b) compounds of the formula

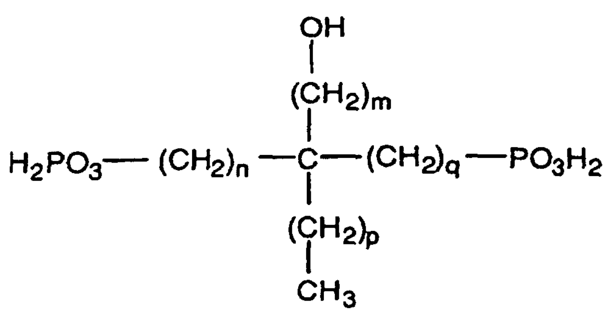

(II)

wherein each of n, m, p, and q is independently 0-4, and water-soluble salts thereof.

**12.** The system of claim 11, wherein said hydrogen peroxide stabilizer is diethylene triamine penta(methylene-phosphonic acid) or a water-soluble salt thereof.

**13.** The system of claim 1, wherein said second composition further comprises a tonicity agent.

**14.** The system of claim 1, wherein said first composition further comprises an enzyme selected from the group consisting of proteases, lipases, and amylases.

## Patentansprüche

1. System zum Desinfizieren und Reinigen einer Kontaktlinse, umfassend:

   (a) eine erste Zusammensetzung, umfassend ein Peroxidaseenzym und ein Jodidsalz;
   (b) eine zweite Zusammensetzung, umfassend eine Wasserstoffperoxidquelle in einer wässrigen Lösung;

   wobei die erste Zusammensetzung in Tablettenform vorliegt, wobei die Tablette Natriumbenzoat und Lactose umfasst; und
   wobei die erste Zusammensetzung weiterhin ein Puffermittel in einer Menge umfasst, die wirksam ist, um Pufferkapazität zum Verhindern einer Absenkung des pH-Werts unter etwa 6,0 bereitzustellen, wenn die erste Zusammensetzung mit der zweiten Zusammensetzung kombiniert wird.

2. System nach Anspruch 1, wobei die erste und zweite Zusammensetzung bis kurz vor Anwendung des Systems getrennt gehalten werden.

3. System nach Anspruch 1, wobei das Puffermittel ausgewählt ist aus der Gruppe, bestehend aus Acetaten, Phosphaten, Boraten, Citraten, Nitraten, Sulfaten, Tartraten, Lactaten, Carbonaten, Bicarbonaten, TRIS, BIS-TRIS-Propan, Goode-Puffern und Gemischen davon.

4. System nach Anspruch 1, wobei die erste Zusammensetzung eine Menge an Jodidsalz umfasst, die ausreicht, um eine Konzentration an Jodidanion von etwa 0,10 bis etwa 5,0 mg/7 ml bereitzustellen, wenn mit der zweiten Zusammensetzung kombiniert.

5. System nach Anspruch 4, wobei die erste Zusammensetzung eine Menge an Jodidsalz umfasst, die ausreicht, um eine Konzentration an Jodidanion von etwa 0,2 bis etwa 1,5 mg/7 ml bereitzustellen, wenn mit der zweiten Zusammensetzung kombiniert.

6. System nach Anspruch 1, wobei die Tablette weiterhin eine Säure und eine Base umfasst, die ein schäumendes Paar bilden, das reagiert, wenn es der zweiten Zusammensetzung ausgesetzt wird.

7. System nach Anspruch 1, wobei das Peroxidaseenzym ausgewählt ist aus der Gruppe, bestehend aus Meerrettichperoxidase, mikrobieller Peroxidase, GD-Peroxidase, Peroxidase SP 540, Lactoperoxidase, Myleroperoxidase und Sojabohnenperoxidase.

8. System nach Anspruch 1, wobei die Wasserstoffperoxidquelle ausgewählt ist aus der Gruppe, bestehend aus Wasserstoffperoxid, Natriumperborat, Natriumperoxid und Harnstoffperoxid.

9. System nach Anspruch 1, wobei die Wasserstoffperoxidquelle Wasserstoffperoxid in einer Menge von 10 ppm bis 1000 ppm, vorzugsweise in einer Menge von 10 ppm bis 100 ppm, bereitstellt.

10. System nach Anspruch 1, wobei die zweite Zusammensetzung weiterhin einen Wasserstoffperoxidstabilisator umfasst.

11. System nach Anspruch 10, wobei der Wasserstoffperoxidstabilisator ausgewählt ist aus der Gruppe, bestehend aus

    (a) Verbindungen der Formel

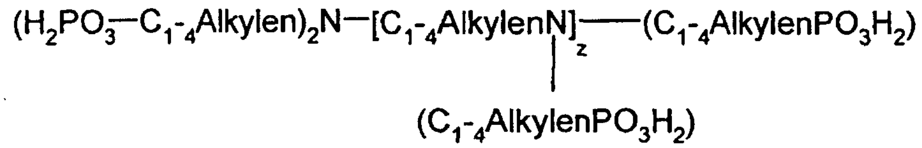

(I)

    worin z eine ganze Zahl von 0-3 ist und in Wasser löslichen Salzen davon; und
    (b) Verbindungen der Formel

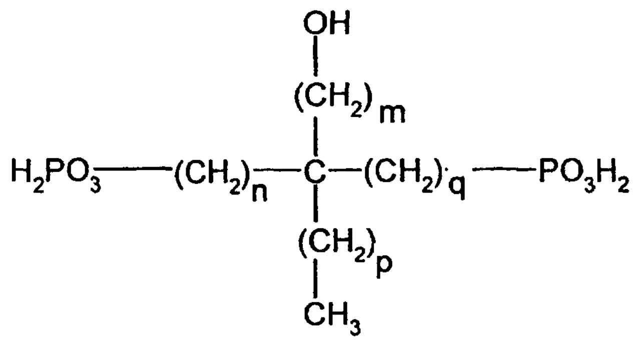
(II)

worin jeder von n, m, p und q unabhängig 0-4 ist und in Wasser löslichen Salzen davon.

**12.** System nach Anspruch 11, wobei der Wasserstoffperoxidstabilisator Diethylentriaminpenta(methylen-phosphonsäure) oder ein in Wasser lösliches Salz davon ist.

**13.** System nach Anspruch 1, wobei die zweite Zusammensetzung weiterhin ein Tonizitätsmittel umfasst.

**14.** System nach Anspruch 1, wobei die erste Zusammensetzung weiterhin ein Enzym, ausgewählt aus der Gruppe, bestehend aus Proteasen, Lipasen und Amylasen, umfasst.

## Revendications

**1.** Système de désinfection et de nettoyage d'une lentille de contact, comprenant :

(a) une première composition comprenant une enzyme peroxydase et un sel d'iodure ;
(b) une seconde composition comprenant une source de peroxyde d'hydrogène en solution aqueuse ;

dans lequel ladite première composition est sous forme de comprimé ;
dans lequel ledit comprimé comprend du benzoate de sodium et du lactose ; et
dans lequel ladite première composition comprend, en outre, un agent tampon en une quantité efficace pour fournir une capacité de tampon pour éviter une diminution du pH à une valeur inférieure à environ 6,0 lorsque ladite première composition est combinée avec ladite seconde composition.

**2.** Système selon la revendication 1, dans lequel lesdites première et seconde compositions sont maintenues séparées juste avant l'utilisation dudit système.

**3.** Système selon la revendication 1, dans lequel ledit agent tampon est choisi dans le groupe constitué par les acétates, les phosphates, les borates, les citrates, les nitrates, les sulfates, les tartrates, les lactates, les carbonates, les bicarbonates, les tampons TRIS, BIS-TRIS-propane, Goode, et leurs mélanges.

**4.** Système selon la revendication 1, dans lequel ladite première composition comprend une quantité de sel d'iodure suffisante pour donner une concentration en anion iodure d'environ 0,10 à environ 5,0 mg/7 ml lorsqu'elle est combinée avec ladite seconde composition.

**5.** Système selon la revendication 4, dans lequel ladite première composition comprend une quantité de sel d'iodure suffisante pour donner une concentration en anion iodure d'environ 0,2 à environ 1,5 mg/7 ml lorsqu'elle est combinée avec ladite seconde composition.

**6.** Système selon la revendication 1, dans lequel ledit comprimé comprend, en outre, un acide et une base qui forment un couple effervescent qui réagit lorsqu'il est exposé à ladite seconde composition.

**7.** Système selon la revendication 1, dans lequel ladite enzyme peroxydase est choisie dans le groupe constitué par la peroxydase de raifort, une peroxydase microbienne, la peroxydase GD, la peroxydase SP 540, l'actoperoxydase, la myléroperoxydase et la peroxydase de soja.

**8.** Système selon la revendication 1, dans lequel ladite source de peroxyde d'hydrogène est choisie dans le groupe constitué par le peroxyde d'hydrogène, le perborate de sodium, le peroxyde de sodium et le peroxyde d'urée.

**9.** Système selon la revendication 1, dans lequel ladite source de peroxyde d'hydrogène donne du peroxyde d'hydrogène en une quantité de 10 ppm à 1000 ppm, de préférence en une quantité de 10 ppm à 100 ppm.

**10.** Système selon la revendication 1, dans lequel ladite seconde composition comprend, en outre, un stabilisant de peroxyde d'hydrogène.

**11.** Système selon la revendication 10, dans lequel ledit stabilisant de peroxyde d'hydrogène est choisi dans le groupe constitué par :

(a) des composés de formule :

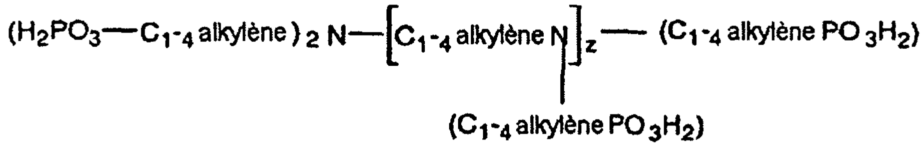

(I)

dans laquelle z est un nombre entier de 0 à 3, et les sels hydrosolubles de ceux-ci, et
(b) des composés de formule :

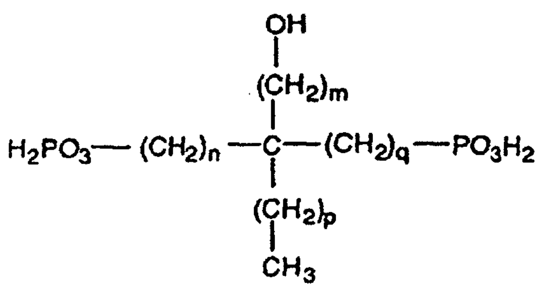

(II)

dans laquelle chacun des indices n, m, p et q a indépendamment une valeur de 0 à 4, et les sels hydrosolubles de ceux-ci.

**12.** Système selon la revendication 11, dans lequel ledit stabilisant de peroxyde d'hydrogène est l'acide diéthylènetriaminepenta(méthylène-phosphonique) ou un sel hydrosoluble de celui-ci.

**13.** Système selon la revendication 1, dans lequel ladite seconde composition comprend, en outre, un agent de tonicité.

**14.** Système selon la revendication 1, dans lequel ladite première composition comprend, en outre, une enzyme choisie dans le groupe constitué par les protéases, les lipases et les amylases.